# EUROPEAN PATENT SPECIFICATION

(11) **EP 4 337 304 B1**
(45) Date of publication and mention of the grant of the patent: **07.10.2026**
(21) Application number: 22723904.3
(22) Date of filing: 27.04.2022
(51) Int. Cl.: A61N 1/36, A61B 5/00, A61N 1/37, A61B 5/311, A61B 5/388

(54) **FEATURE EXTRACTION WITH AUTOMATIC SAMPLING WINDOW DEFINITION**
MERKMALSEXTRAKTION MIT AUTOMATISCHER ABTASTFENSTERDEFINITION
EXTRACTION DE CARACTÉRISTIQUES AVEC DÉFINITION AUTOMATIQUE DE FENÊTRE D'ÉCHANTILLONNAGE

(30) Priority: 10.05.2021 US 202163186577 P
(43) Date of publication of application: 20.03.2024
(73) Proprietor: 1/1Boston Scientific Neuromodulation Corporation, Valencia, CA 91355 (US)
(72) Inventor: WEISS, Philip Leonard, Sherman Oaks, California 91401 (US); ESTELLER, Rosana, Santa Clarita, California 91390 (US); JAYAKUMAR, Adarsh, Valencia, California 91355 (US); UYEDA, Joshua, Los Angeles, California 90066 (US); ZHANG, Tianhe, Studio City, California 91604 (US)
(74) Representative: Vossius & Partner Patentanwälte Rechtsanwälte mbB
(86) International application number: PCT/US2022/026528
(87) International publication number: WO 2022/240587

(56) References cited:
- US-A1- 2003 195 580
- US-A1- 2007 225 767
- US-A1- 2019 209 844
- US-A1- 2019 298 992
- US-A1- 2020 155 019
- US-A1- 2020 305 745

## Description

### CLAIM OF PRIORITY

This application claims the benefit of U.S. Provisional Application No. 63/186,577, filed on May 10, 2021.

### TECHNICAL FIELD

This document relates generally to medical systems, and more particularly, but not by way of limitation, to systems, devices, and methods for extracting features from a sensed signal.

### BACKGROUND

Various therapies may deliver electrical energy to a patient. Examples of such therapies include, but are not limited to, muscle stimulators, cardiac rhythm devices such as pacemakers and defibrillators, and neurostimulators. Physiological signal(s) may be sensed for various reasons related to the delivered therapy, such as to time the therapy delivery, to determine enabling or disabling conditions for delivering the therapy, to determine an efficacy of a therapy, or to provide feedback for closed-loop control of the therapy. For example, action potentials within a nerve may be sensed to provide closed-loop control of a neuromodulation therapy. Examples of neuromodulation include Spinal Cord Stimulation (SCS), Deep Brain Stimulation (DBS), Peripheral Nerve Stimulation (PNS), and Functional Electrical Stimulation (FES).
US 2003/0195580 A1 discloses a cardiac stimulation device and method measure a myo-cardial conduction time and store its long-term running average. A multipolar lead is used to deliver a stimulation pulse from a tip electrode and detect the evoked response after it has propagated to a ring electrode. The time between the stimulation pulse and a detected feature of the evoked response is determined as the myocardial conduction time. A long-term average myocardial conduction time is calculated by averaging on the order of five hundred stimulated cardiac cycles, and a running average is stored in memory. The effect of heart rate on myocardial conduction may be taken into account by storing multiple averages corresponding to multiple pacing rates or storing the conduction time average as a function of heart rate. The effect of pacing pulse energy on measured myocardial conduction may be taken into account by storing multiple averages corresponding to multiple pacing rates or storing the conduction time average as a function of pacing pulse energy. Shifts in the myocardial conduction time may be used for monitoring disease progression or the long-term response to a treatment.

### SUMMARY

The invention is defined by the features of the independent claim. Preferred embodiments are defined by the dependent claims.

Aspects, examples, embodiments and methods described hereinafter are of exemplary nature and are presented for better understanding the present invention which is defined by the appended claims.

This Summary is an overview of some of the teachings of the present invention and the present disclosure, but other aspects of the disclosure will be apparent to persons skilled in the art upon reading and understanding the following detailed description and viewing the drawings that form a part thereof, each of which are not to be taken in a limiting sense. The scope of the present invention is defined by the appended claims.

### BRIEF DESCRIPTION OF THE DRAWINGS

Various embodiments are illustrated by way of example in the figures of the accompanying drawings. Such embodiments are demonstrative and not intended to be exhaustive or exclusive embodiments of the present disclosure.
FIG. 1 illustrates, by way of example, an embodiment of a neuromodulation system.
FIG. 2 illustrates, by way of example and not limitation, the neuromodulation system of FIG. 1 implemented in a spinal cord stimulation (SCS) system or a deep brain stimulation (DBS) system.
FIG. 3 illustrates, by way of example and not limitation, an embodiment of a modulation device, such as may be implemented in the neuromodulation system of FIG. 1, that includes sensing circuitry.
FIG. 4 is a diagram illustrating a relationship between a stimulation electrode and a sensing electrode.
FIG. 5 illustrates, by way of example and not limitation, a method of extracting features from sensed electrical potentials for use in providing feedback for closed-loop control of an electrical therapy.
FIG. 6 illustrates, by way of example and not limitation, feature extraction potentially corrupted by stimulation artifacts.
FIG. 7 illustrates, by way of example and not limitation, a sampling window for detecting features and excludes a stimulation artifact.
FIG. 8A illustrates, by way of example and not limitation, a stimulation artifact within the sampling window which may potentially cause corruption in the detection of the signal features, and FIG. 8B illustrates an automatic adjustment of the sampling window to exclude the stimulation artifact.
FIG. 9A illustrates, by way of example and not limitation, a sampling window whose timing fails to correspond to the timing of the features that are desired to be detected in the signal, and FIG. 9B illustrates an automatic adjustment of the timing for the sampling window to correspond to the timing of the features to be detected in the signal.
FIG. 10 illustrates, by way of example, a process for verifying that the timing of the features that are desired to be detected in the signal fall outside of the timing for the sampling window before automatically adjusting the timing of the sampling window.
FIG. 11 illustrates, by way of example and not limitation, a method for moving the timing for the sampling window away from a stimulation artifact by detecting both the stimulation artifact and features in the signal and comparing the features to expected features.
FIG. 12 illustrates an example of a look-up table for presetting a sampling window based on estimated conduction velocities and distance between stimulation and sensing electrodes.
FIG. 13 illustrates, by way of example and not limitation, a method that compares features in different sensing channels to detect the features and set the timing of the sampling window.
FIG. 14 illustrates, by way of example and not limitation, signals in two different sensing channels that may be compared to detect the features in the signal and set the timing of the sampling window.

### DETAILED DESCRIPTION

The following detailed description refers to the accompanying drawings which show, by way of illustration, specific aspects and exemplary embodiments in which the present disclosure may be practiced. These embodiments are described in sufficient detail to enable those skilled in the art to practice the present disclosure. References to "an", "one", or "various" embodiments in this disclosure are not necessarily to the same embodiment, and such references contemplate more than one embodiment. The following detailed description is, therefore, not to be taken in a limiting sense, and the scope is defined only by the appended claims.

The present disclosure relates to extracting features from a sensed signal. A programmable timing window may be used to extract features from sensed signals. The sensed signals may be indicative of physiological activity such as may be produced by a variety of physiological sensors. By way of example and not limitation, the sensed physiological activity may include muscle activity or neural activity. Examples of neural activity may include, but are not limited to, neural activity in a brain activity, spinal cord and/or peripheral nerve(s). Sensing of neural activity is described herein as a more specific application. Those of ordinary skill in the art would understand, upon reading and comprehending this disclosure, how to apply the teachings herein toward other sensed signals. The sensed signals may be indicative of evoked compound action potentials (ECAPs), evoked resonant neural activity (ERNA), or local field potentials (LFPs)).

Feature(s) may be extracted from the sensed signal. However, the extracted feature may be corrupted if part of a stimulation artifact occurs inside the sensing time window, such as may happen after a stimulation parameter change (e.g., amplitude). Embodiments described herein automatically overcome this potential issue. By way of an example and not limitation, when an extracted feature has a significant change, the present disclosure may use an automatic peak detection block to correlate its peak-detection finding with the extracted features. For example, the extracted feature may be a range (maximum-minimum). The maximum (or minimum) may be typically located within a range of samples (e.g., samples 32-37). After a stimulation amplitude adjustment, the maximum in the sensing window may make a significant move away from the typical locations (e.g. the maximum may move to sample 3). The CPU may be interrupted and the firmware may look at the automatic peak detected to see if there is a peak found close to the typical locations (e.g., samples 32-37). If there is a peak found close to the typical locations, then it can be determined that the peak found at sample 3 is an artifact. The sensing window may be tightened to avoid the maximum from the stimulation artifact by, for example, starting the sensing window at sample 5. If no peak is found close to the typical location, then the system can wait until the next stimulation period to reassess. This reassessment may continue when no peak is found until the system triggers storage of raw data (internal trigger), so the user can reassess if the features are operating correctly.

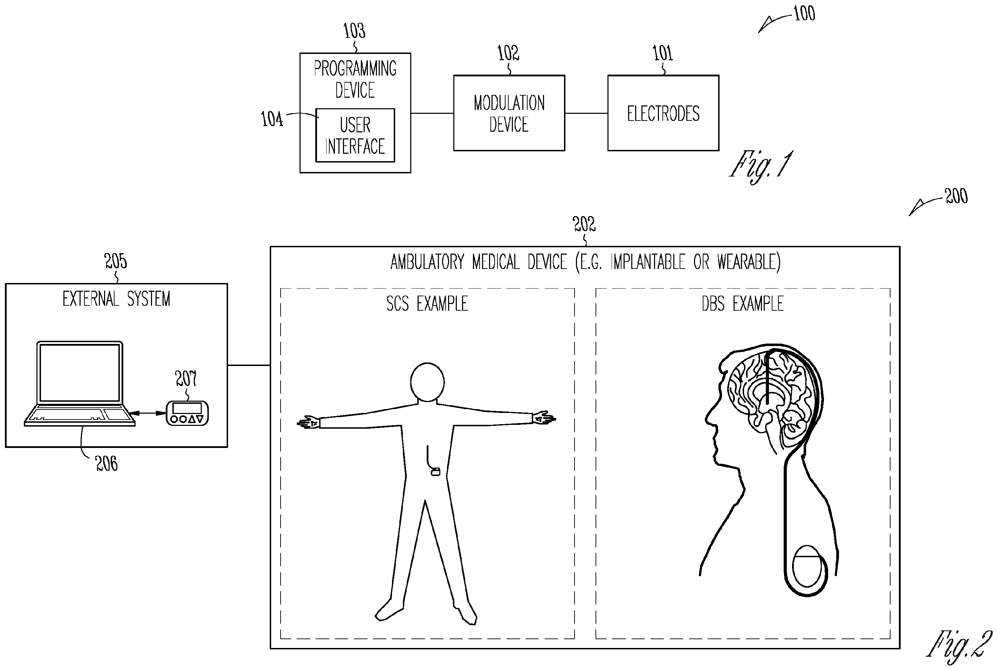

FIG. 1 illustrates, by way of example, an embodiment of a neuromodulation system. The illustrated neuromodulation system 100 includes electrodes 101, a modulation device 102, and a programming system such as a programming device 103. The programming system may include multiple devices. The electrodes 101 are configured to be placed on or near one or more neural targets in a patient. The modulation device 102 is configured to be electrically connected to electrodes 101 and deliver neuromodulation energy, such as in the form of electrical pulses, to the one or more neural targets though electrodes 101. The system may also include sensing circuitry to sense a physiological signal, which may but does not necessarily form a part of modulation device 102. The delivery of the neuromodulation is controlled using a plurality of modulation parameters that may specify the electrical waveform (e.g. pulses or pulse patterns or other waveform shapes) and a selection of electrodes through which the electrical waveform is delivered. In various embodiments, at least some parameters of the plurality of modulation parameters are programmable by a user, such as a physician or other caregiver. The programming device 103 provides the user with accessibility to the user-programmable parameters. The programming device 103 may also provide the use with data indicative of the sensed physiological signal or feature(s) of the sensed physiological signal. In various embodiments, the programming device 103 is configured to be communicatively coupled to modulation device via a wired or wireless link. In various embodiments, the programming device 103 includes a user interface 104 such as a graphical user interface (GUI) that allows the user to set and/or adjust values of the user-programmable modulation parameters. The user interface 104 may also allow the user to view the data indicative of the sensed physiological signal or feature(s) of the sensed physiological signal and may allow the user to interact with that data.

FIG. 2 illustrates, by way of example and not limitation, the neuromodulation system of FIG. 1 implemented in a spinal cord stimulation (SCS) system or a deep brain stimulation (DBS) system. The illustrated neuromodulation system 200 includes an external system 205 that may include at least one programming device. The illustrated external system 205 may include a clinician programmer 206 configured for use by a clinician to communicate with and program the neuromodulator, and a remote control 207 configured for use by the patient to communicate with and program the neuromodulator. For example, the remote control device may allow the patient to turn a therapy on and off and/or may allow the patient to adjust patient-programmable parameter(s) of the plurality of modulation parameters. FIG. 2 illustrates a modulation device as an ambulatory medical device 202. Examples of ambulatory devices include wearable or implantable neuromodulators.

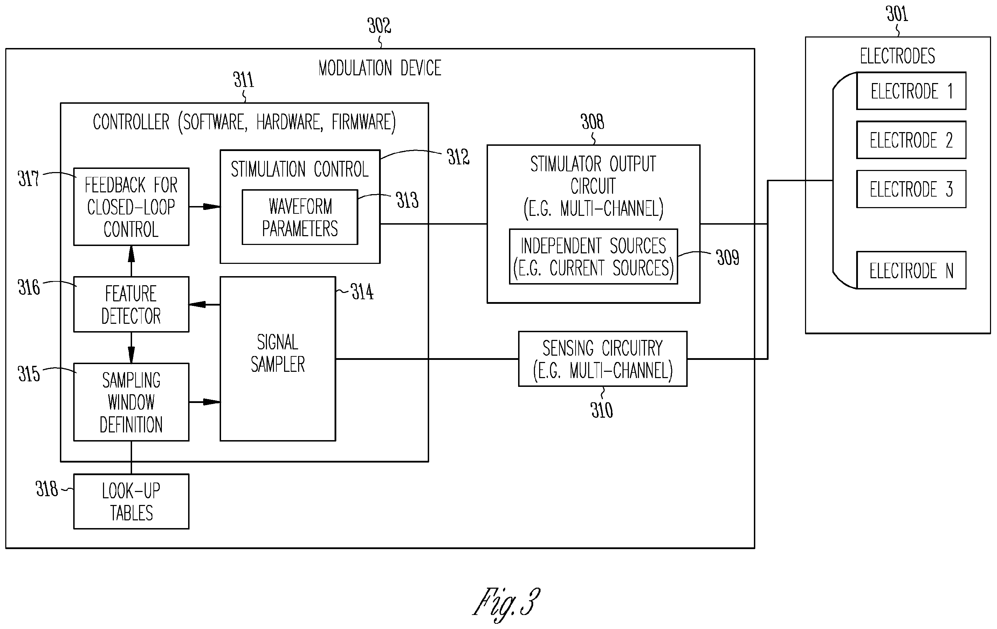

FIG. 3 illustrates, by way of example and not limitation, an embodiment of a modulation device, such as may be implemented in the neuromodulation system of FIG. 1, that includes sensing circuitry. The modulation device 302 may be configured to be connected to electrode(s) 301, illustrated as N electrodes. Any one or more of the electrodes 301 may be configured for use to deliver modulation energy, sense electrical activity, or both deliver modulation energy and sense electrical activity. The modulation device 302 may include a stimulator output circuit 308 configured to deliver modulation energy to electrode(s). The stimulator output circuit 308 may be configured with multiple (e.g., two or more) channels for delivering modulation energy, where each channel may be independently controlled with respect to other channel(s). For example, the stimulator output circuit 308 may have independent sources 309 such as independent current sources or independent voltage sources. The modulation device 302 may include sensing circuitry 310 configured to receive sensed electrical energy from the electrode(s), such as may be used to sense electrical activity in neural tissue or muscle tissue. The sensing circuitry may be configured to process signals in multiple (e.g., two or more) channels. By way of example and not limitation, the sensing circuitry 310 may be configured to amplify and filter the signal(s) in the channel(s). Additionally or alternatively, the sensing circuitry may be configured for use with other types physiological sensors.

The modulation device 302 may include a controller 311 operably connected to the stimulation output circuit 308 and the sensing circuitry 310. The controller may include a stimulation control 312 configured for controlling the stimulator output circuit 308. For example, the stimulation control 312 may include start / stop information for the stimulation and/or may include relative timing information between stimulation channels. The stimulation control 312 may include waveform parameters that control the waveform characteristics of the waveform produced by the stimulation output circuit 308. The waveform parameters 313 may include, by way of example and not limitation, amplitude, frequency, and pulse width parameters. The waveform parameters may include, by way of example and not limitation, regular and./or irregular patterns of pulses. The waveform parameters may, but does not necessary, define more than one waveform shape. The stimulation control 312 may be configured to change waveform parameter(s) (e.g., one or more waveform parameters) in response to user input and/or automatically in response to feedback.

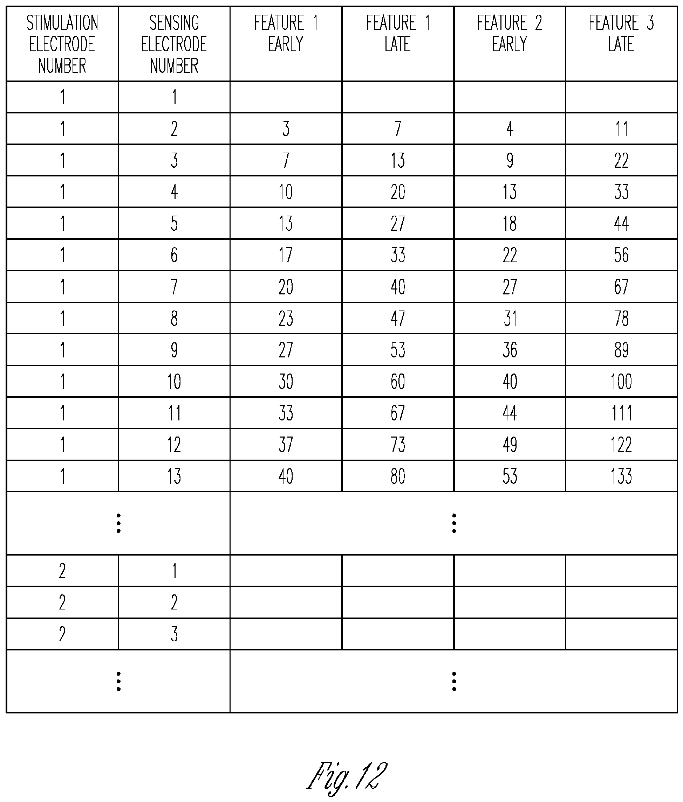

The controller 311 may include a signal sampler 314 configured for use to sample a signal produced by the sensing circuitry 310. The signal may be sampled within a sampling window. The controller 311 may further includes a sampling window definition 315 for defining timing of the sampling window, and a feature detector 316 configured to detect one or more features in the sampled signal. The timing of the sampling window after a pulse may be based on the timing of the pulse. For example, the pulse window may be timed to begin a programmable period of time after the pulse and last for a programmable period of time or end a programmable period of time after the pulse. Examples of features that may be detected include peaks (e.g., minimum and/or maximum peaks including local peaks/inflections), range between minimum / maximum peaks, local minima and/or local maxima, area under the curve (AUC), and curve length between points in the curve. Detected feature(s) from the feature detector 316 may be used as feedback for closed-loop control 317 of the therapy. The closed-loop control 317 may be used by the stimulation control 312 to adjust the stimulation (e.g., parameter(s)). In some embodiments, the modulation device 302 may include look-up table(s) 318 that include estimated timing values for various combinations of stimulation and sensing electrodes, as discussed below with respect to FIG. 12. The look-up table(s) may be used to set initial timing for the sampling window.

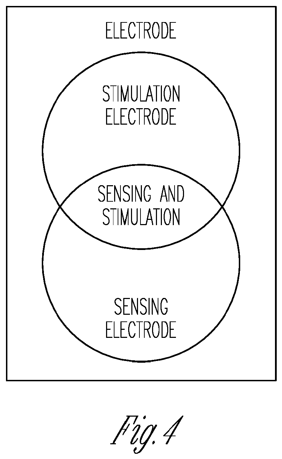

FIG. 4 is a diagram illustrating a relationship between a stimulation electrode and a sensing electrode. The stimulation electrode is configured for use in delivering modulation energy, and the sensing electrode is configured for use in sensing electrical activity. As illustrated by FIG. 4, the stimulation electrode may also be used in sensing electrical activity, and the sensing electrode may also be used in delivering modulation energy. Thus, the term "stimulation electrode" does not necessary exclude the electrode from also being used to sense electrical activity; and the term "sensing electrode" does not necessarily exclude the electrode from also being used to deliver modulation energy.

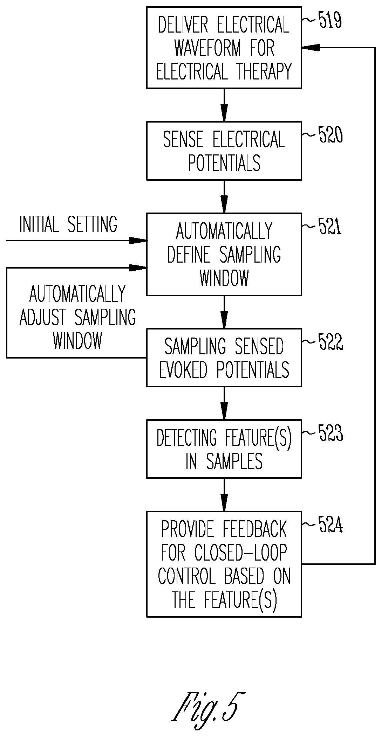

FIG. 5 illustrates, by way of example and not limitation, a method of extracting features from sensed electrical potentials for use in providing feedback for closed-loop control of an electrical therapy. At 519, an electrical waveform for an electrical therapy is delivered. For example, the electrical waveform may be delivered using a stimulator output circuit 308 such as illustrated in FIG. 3. Electrical potentials are sensed at 520. The electrical potentials may be sensed using sensing circuitry 310 such as illustrated in FIG. 3. A sampling window may be automatically defined at 521, and the sensed evoked potentials may be sampled using the defined sampling window at 522. For example, the sampling window definition 315 illustrated in FIG. 3 may be used to automatically define the sampling window. The sampling window may be defined with initial settings, such as determined using look-up table(s) 318 and/or user input(s), and/or may be automatically adjusted based on the sensed samples, such as is illustrated at 314, 316, 315 in FIG. 3. Feature(s) in the sample(s) may be detected 523, and feedback for closed-loop control of the therapy may be provided based on the detected feature(s) 524. The feature(s) may be detected at 316 and the feedback may be provided at 317 in FIG. 3. Examples of features that may be detected include, but are not limited to, peaks (e.g., minimum and/or maximum peaks including local peaks/inflections), range between minimum / maximum peaks, local minima and/or local maxima, area under the curve (AUC), and curve length between points in the curve.

The sampling window may be automatically defined based on an estimated conduction velocity for the evoked potentials and a distance between the stimulation electrode and the sensing electrode. For example, a lookup table may be used to define the sampling window. The lookup table may identify a start and an end of the sampling window for a selected stimulation electrode and a selected sensing electrode from a plurality of electrodes.

The sampling window may be automatically defined based on specific feature(s) associated with specific time(s) in the evoked responses. By way of example and not limitation, the specific feature(s) associated with specific time(s) may be, for a particular time window, one or more of an area under the curve, a curve length of the signal for the particular time window, a range (maximum minus minimum), oscillation frequency, or rate of decay in amplitude of peak(s), difference of any two positive and negative peak magnitudes, or change of any of the values for any of these features with respect to a baseline feature value.

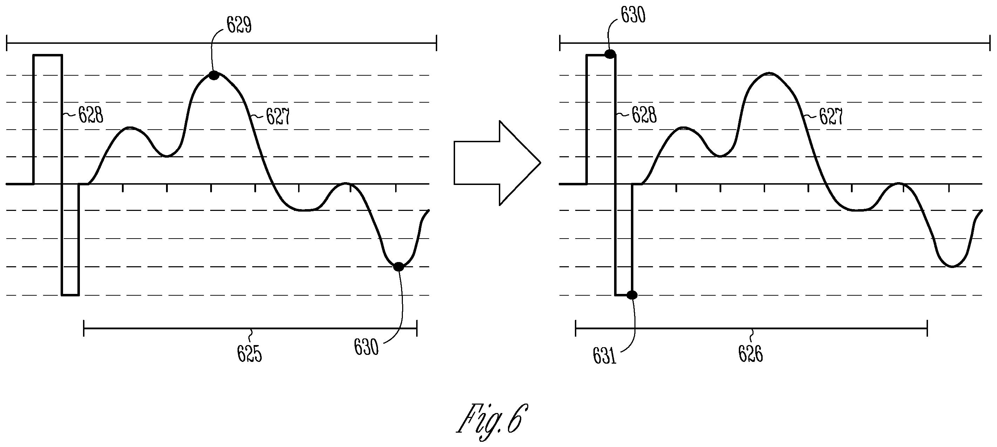

FIG. 6 illustrates, by way of example and not limitation, feature extraction potentially corrupted by stimulation artifacts. A first sampling window and a second sampling window are illustrated along with a signal and stimulation artifact. FIG. 6 illustrates that, when the first sampling window follows the stimulation artifact, the maximum feature and the minimum feature may be accurately detected. However, when the second sampling window includes the stimulation artifact, the artifact may cause the system to find an incorrect maximum feature 631 and an incorrect minimum feature 631. The timing of the stimulation artifact may change because, by way of example and not limitation, a change in a stimulation parameter such as amplitude or lead / electrode migration. The effect may be that the artifact 628 occurs within the sensing window 626.

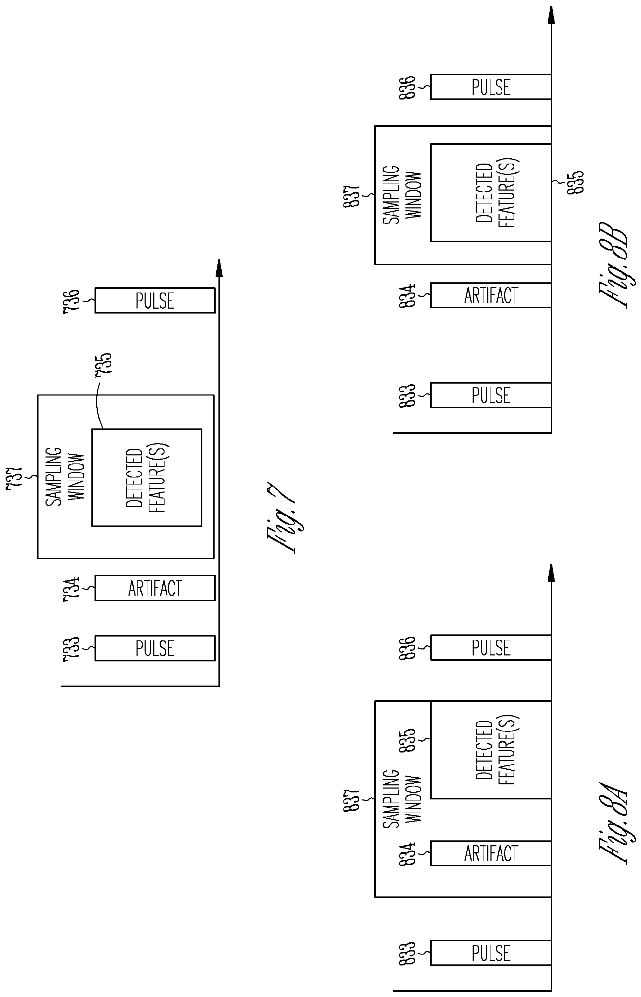

FIG. 7 illustrates, by way of example and not limitation, a sampling window for detecting features and excludes a stimulation artifact. The illustration includes a stimulation pulse 733, a stimulation artifact 734 within the signal, detected features within the signal 735, and the subsequent pulse 736. The sampling window 737 is properly timed to begin after the artifact 737 and before the subsequent pulse 736 to be able to accurately detect feature(s) 735 within the signal.

FIG. 8A illustrates, by way of example and not limitation, a stimulation artifact within the sampling window which may potentially cause corruption in the detection of the signal features, and FIG. 8B illustrates an automatic adjustment of the sampling window to exclude the stimulation artifact. Similar to FIG. 7, these figures show a stimulation pulse 833, a stimulation artifact 834 within the signal, detected features 835 within the signal, and the subsequent pulse 836. However, in FIG. 8A, the timing of the sampling window 837 overlaps the artifact 834, which may cause errors in detecting the features. The present disclosure may automatically adjust the sampling window 837 to start after artifact 834 and end before the subsequent pulse 836 as illustrated in FIG. 8B, to accurately detect the features 835 without corruption from the artifact 834.

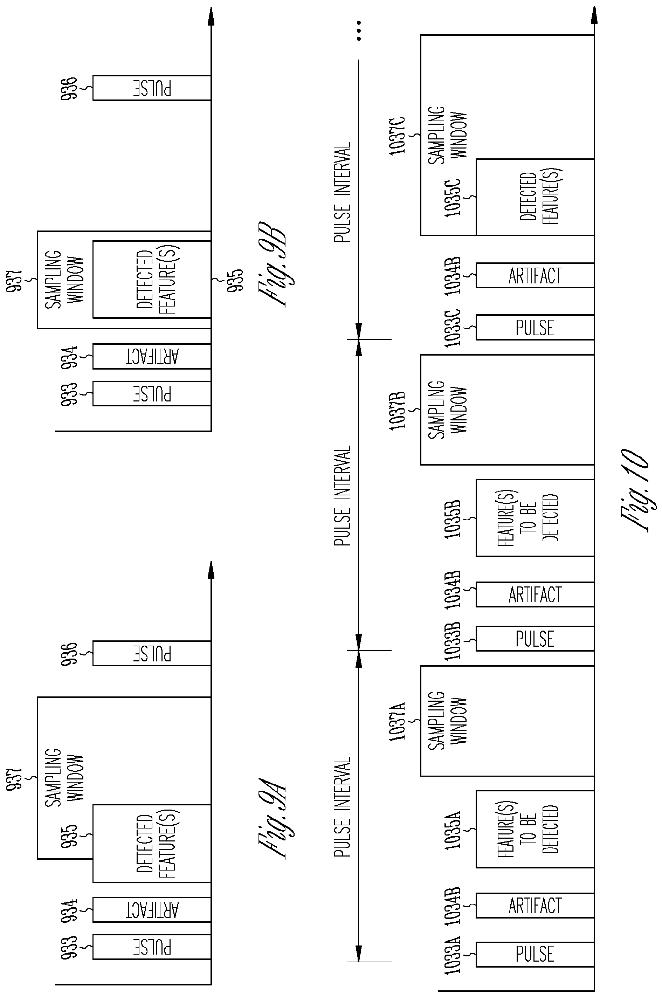

FIG. 9A illustrates, by way of example and not limitation, a sampling window whose timing fails to correspond to the timing of the features that are desired to be detected in the signal, and FIG. 9B illustrates an automatic adjustment of the timing for the sampling window to correspond to the timing of the features to be detected in the signal. Similar to FIG. 7, these figures show a stimulation pulse 933, a stimulation artifact 934 within the signal, detected features 935 within the signal, and the subsequent pulse 936. However, in FIG. 9A, the timing of the sampling window 937 fails to capture at least part of the feature(s) causing errors in detecting the features. The present disclosure may automatically adjust the sampling window 937 to move and/or resize the sampling window while still starting after artifact 934 and ending before the subsequent pulse 936 as illustrated in FIG. 9B, to accurately detect the features 935 without corruption from the artifact 934.

Thus, the controller may automatically adjust the sampling window (e.g., starting earlier and/or later and/or ending earlier and/or later) during the course of the electrical therapy. The sampling window may be adjusted to avoid a stimulation artifact from interfering with detecting the at least one feature (e.g., at least one of a minimum or a maximum in the sensed evoked potentials or other feature). The controller may determine whether the at least one feature occurs before an expected period of time or a number of samples after the stimulation pulse and whether another feature occurs during the expected period of time or the number of samples after the stimulation pulse, and the controller may be configured to delay the beginning of the sampling window after the stimulation pulse.

FIG. 10 illustrates, by way of example, a process for verifying that the timing of the features that are desired to be detected in the signal fall outside of the timing for the sampling window before automatically adjusting the timing of the sampling window. The figure illustrates a series of stimulation pulses 1033A, 1033B and 1033 C, corresponding stimulation artifacts 1034A, 1034B, 1034C within the signal, corresponding signal features 1035A, 1035B, 1035C, and sampling windows 1037A. The pulse-to pulse timing may be referred to pulse intervals. The pulse intervals may be equal (e.g., pulse period) as would be delivered using a single pulse frequency, or may be unequal as may be delivered using irregular pulse patterns. As illustrated, the feature(s) to be detected 1035A occur earlier then the sampling window, such that the features will fail to be detected within the sampling window 1037A. Before adjusting the sampling window, the system may be configured to evaluate to confirm in one or more pulse intervals that the features are not being detected. Thus, in the subsequent pulse interval, for example, it may be determined the features 1035B are not detected within sampling window 1037B. After failing to detect features in one or more subsequent pulse intervals, the process may adjust the beginning of the sampling window 1037C to be earlier in order to be able to detect the features 1035C. Thus, before automatically adjusting the sampling window, the system may reassess over the course of one or more additional pulse intervals whether the at least one feature occurs before or after and does not occur during the expected period of time or sample range after the stimulation pulse.

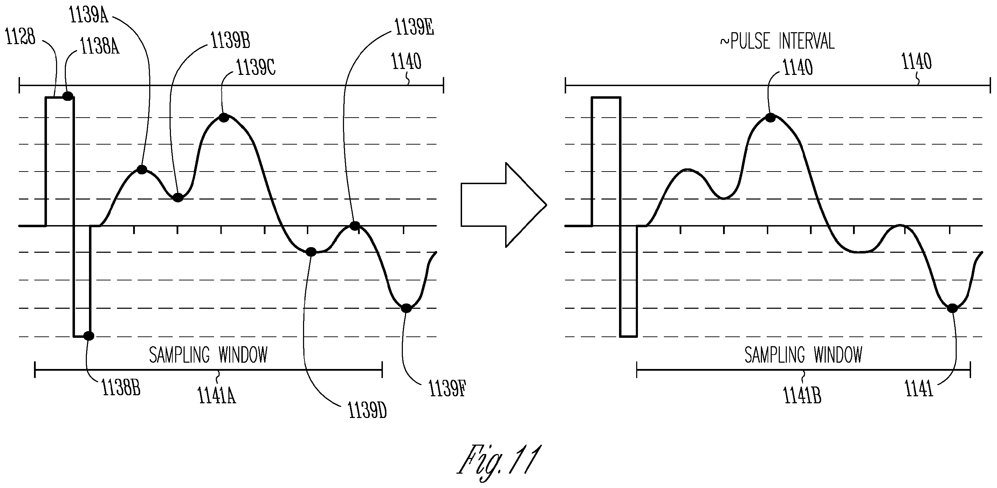

FIG. 11 illustrates, by way of example and not limitation, a method for moving the timing for the sampling window away from a stimulation artifact by detecting both the stimulation artifact and features in the signal and comparing the features to expected features. The signal over a duration of about a pulse interval is illustrated. Each of the peaks (e.g., local minima and local maxima) may be detected in the signal during the sampling window 1141A, including that maximum 1138A and minimum 1138B from the stimulation artifact 1138A and peaks within the signal 1139A-1139F during the sampling window 1141A. The system can be configured to find the expected peak 1139C, and expected trough 1139F from within the found peaks 1139A-1139F within the signal, and then automatically move the programmable sampling window away from the artifact (e.g., moving from sampling window 1141A to sampling window 1141B). Thus, the controller may find an expected peak and an expected trough in the sensed evoked potentials, and use the expected peak and the expected trough as a reference to move the sampling window to avoid the stimulation artifact. This automatic adjustment can performed when the feature computed is peaks or range, as well as other features such as Curve Length or Area Under the Curve (AUC). The automatic adjustment of the sampling window allows the system to avoid corrupted feature values.

The automatic adjustment can also be used to determine the initial placement of the sample window without the need for external user interaction. A look-up table based on estimated conduction velocities, stim-rec electrode distance /electrode design/inter-contact spacing, and artifact features may be used to preset the sampling window. For example, a look-up table may be based on distance to velocity conversion formula that accounts for lead spacing. The table may be configurable and/or lead specific. The windows may be based on time (e.g., time windows) or based on samples (e.g., sample windows). For example, time windows may be defined by v = d/t; t = v/d, where "v" is velocity, "d" is distance, "t" is time. The table can be created for a range of potential velocities (fastest / slowest). Sample windows may be defined by s = (time per sample * v)/d, where "s" is samples.

Conduction velocities may be pre-loaded or pre-configured. The windows may be then set according to these pre-established velocities. The conduction velocities may be pre-defined from external sources such as, but not limited to academic literature or cloud data and/or the conduction velocities may be pre-loaded as a default. The pre-loaded conduction velocities may be used to configure the look-up table. The look-up table itself may be pre-defined by lead type and contact separation. A fixed default window may be deployed in the absence of other information.

Thus, the controller may automatically define the sampling window based on an estimated conduction velocity for the evoked potentials and a distance between the stimulation electrode and the sensing electrode, and may use a lookup table to define the sampling window. The lookup table may identify a start and an end of the sampling window for a selected stimulation electrode and a selected sensing electrode from a plurality of electrodes.

By way of example and not limitation, the sampling window may be automatically defined based on specific feature(s) associated with specific time(s) in the evoked responses. By way of example and not limitation, the specific feature(s) associated with specific time(s) may be, for a particular time window, one or more of an area under the curve, a curve length of the signal for the particular time window, a range (maximum minus minimum), oscillation frequency, or rate of decay in amplitude of peak(s), difference of any two positive and negative peak magnitudes, or change of any of the values for any of these features with respect to a baseline feature value.

FIG. 12 illustrates an example of a look-up table for presetting a sampling window based on estimated conduction velocities and distance between stimulation and sensing electrodes. Each potential stimulation electrode / sensing electrode combination may be listed on the side. For example, for sixteen electrodes, there may be row for each stimulation electrode corresponding to each of the sixteen sensing electrodes. For each stimulation electrode / sensing electrode combination, the row may include limits (e.g., early / late) for a range of values within which a feature is expected to occur. The illustrated table includes two features, and a value for an early limit and a late limit for each of features. The sampling window (e.g., initial sampling window) may be set based on the stimulation / sensing configuration and the estimated early and late times for each feature for that stimulation / sensing configuration. The latencies and time windows in the look up table may depend on the make and model of the lead and/or the distance separations between contacts. For example, a larger sampling window difference may be implemented to accommodate more distant contacts.

Optionally, an artifact exclusion interval may be added. Artifacts tend to be quasistatic with stimulation and will have fixed width following stimulation. For example, the exclusion interval may be based on the pulse width of a pulse and/or may be include an offset period of time. By way of a more specific example, the exclusion interval may be two times the pulse width summed with 200 µs for active recharge.

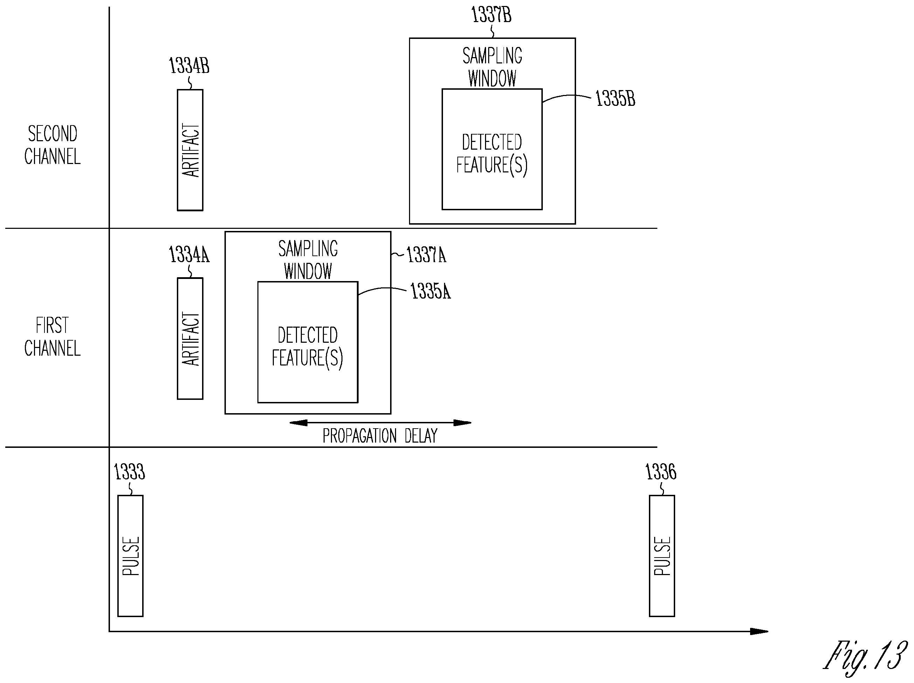

FIG. 13 illustrates, by way of example and not limitation, a method that compares features in different sensing channels to detect the features and set the timing of the sampling window. The method takes advantage of ECAP signal propagation vs. quasistatic artifact by finding that peaks in two channels rostrocaudally-oriented channels indicate an artifact in the signal if the peaks are equally timed. The peaks may also indicate an artifact if the peak on a channel farther from the stimulation channel occurs before the peak on the channel closer to the stimulation channel.

Physiological signals will likely exhibit propagation, showing an appreciable delay on multiple channels. A sampling window may be established based on the first set of corresponding peaks in the channels where such propagation is observed (e.g., 0.1 ms or the like).

FIG. 13 illustrates a stimulation pulse 1333 and a subsequent stimulation pulse 1336. Two sensing channels may sense the artifact substantially at the same time, as illustrated at 1334A and 1334B. However, as the physiological signal exhibits a propagation delay, the feature(s) 1335A and 1335B to be detected within the channels will also exhibit a propagation delay. The sampling window may be adjusted to detected the features, such as based on the similar first peaks in the physiological signal that exhibit the propagation delay. Thus, the sensing circuitry may sense on a first channel using at least a first electrode at a first location and sense on a second channel using at least a second electrode at a second location. The controller may be configured to sample the sensed evoked potentials on each of the first and second channels detect at least one feature on each of the channels, and automatically determine that the at least one feature on the first channel corresponds to the at least one feature on the second channel with an expected propagation delay from the first location to the second location.

Some embodiments may sense signals in more than two different sensing channels. In some embodiments, the use of additional sensing channels beyond two sensing channels may provide greater confidence of the expected propagation. For example, four sensing channels may be used to identify the expected propagation delay. In some embodiments, the use of additional sensing channels may enable other expected propagation delays to be identified. For example, some of the sensing channels (e.g., channels 0 and 1) may be used to identify an expected propagation delay and other ones of the sensing channels (e.g., channels 2 and 3) may be used to identify another, different expected propagation delay in the signal.

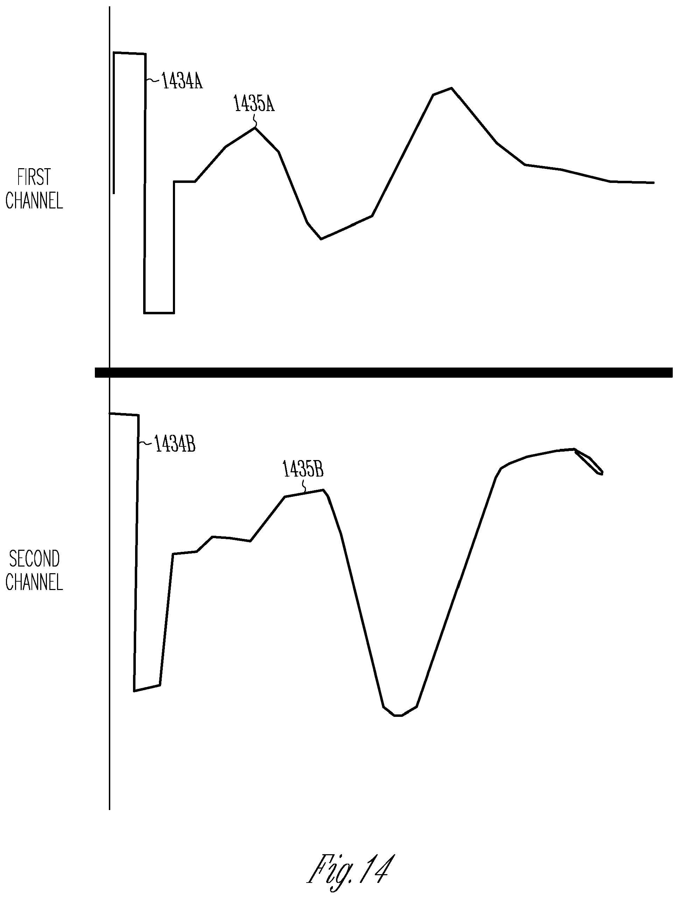

FIG. 14 illustrates, by way of example and not limitation, signals in two different sensing channels that may be compared to detect the features in the signal and set the timing of the sampling window. The artifact 1434A and 1434B sensed in the two channels occurs near simultaneously, whereas the physiological signal in the channels exhibit a signal delay. For example, a peak 1435B in the second channel is similar to the peak 1435A in the first channel with a propagation delay. The sampling window may be set based on first peak 1435A and 1435B that illustrates such propagation.

Some features, such as artifact features, may not display any latency at all between channels. That is, the feature does not just occur outside of the expected window, the feature may not change latencies at all across channels 1, 2, and beyond). Some embodiments may essentially eliminate any feature that does not exhibit any latency changes beyond a small variance (e.g., 1-2 samples) among multiple channels, particularly if the feature is outside of the window.

Some embodiments may implement an algorithm to provide an automated initial placement of the sample window. Similar to FIGS. 8A-8B, the algorithm may place the window at a set point in time after the stimulation pulse that would necessarily include the artifact and evoked potential, such as may be represented by FIG. 8A. The algorithm may move the sample window back in time to exclude the artifact and / or center the window on the evoked potential, such as may be represented by FIG. 8B. The algorithm may include bounds on the expected amplitude and time range of the evoked potential to ensure the "found" feature (e.g., peak and/or trough) is the desired evoked potential. This algorithm may be used to enable sensing for a particular therapy program without needing a user to calibrate the precise position of the sensing window. Such an algorithm may be useful because sensing window position may besensitive to variations in impedance, lead placement / migration, and other physiological changes, and because a patient can have many different sensing enabled therapy programs some of which may not be run for a relatively long time after initial programming.

The above detailed description includes references to the accompanying drawings, which form a part of the detailed description. The drawings show, by way of illustration, specific embodiments in which the invention may be practiced. These embodiments are also referred to herein as "examples."

Method examples described herein may be machine or computer-implemented at least in part. Some examples may include a computer-readable medium or machine-readable medium encoded with instructions operable to configure an electronic device to perform methods as described in the above examples. An implementation of such methods may include code, such as microcode, assembly language code, a higher-level language code, or the like. Such code may include computer readable instructions for performing various methods. The code may form portions of computer program products. Further, in an example, the code may be tangibly stored on one or more volatile, non-transitory, or non-volatile tangible computer-readable media, such as during execution or at other times. Examples of these tangible computer-readable media may include, but are not limited to, hard disks, removable magnetic disks, removable optical disks (e.g., compact disks and digital video disks), magnetic cassettes, memory cards or sticks, random access memories (RAMs), read only memories (ROMs), and the like.

The above description is intended to be illustrative, and not restrictive. Other embodiments may be used, such as by one of ordinary skill in the art upon reviewing the above description. The scope of the invention should be determined with reference to the appended claims.

## Claims

1. A system (100, 200), comprising:
a stimulator (102, 302) operably connected to at least one stimulation electrode (101, 301), and configured to deliver an electrical waveform for an electrical therapy using the at least one stimulation electrode;
sensing circuitry (310) operably connected to at least one sensing electrode, and configured to sense electrical potentials that are evoked by the electrical waveform to provide sensed evoked signals;
a controller (311) operably connected to the stimulator and the sensing circuitry (310), wherein the controller is configured to
automatically define a sampling window;
sample the sensed evoked potentials during the sampling window to provide sampled values;
detect at least one feature from the sampled values; and
automatically provide feedback for closed-loop control of the electrical therapy based on the at least one feature,
wherein the controller (311) is configured to automatically define the sampling window by:
using a lookup table to automatically define the sampling window based on an estimated conduction velocity for the evoked potentials and a distance between the stimulation electrode and the sensing electrode, the lookup table identifying a start and an end of the sampling window for a selected stimulation electrode and a selected sensing electrode from a plurality of electrodes.

2. The system (100, 200) of claim 1, wherein the sensing circuitry (310) is configured to sense on at least two channels, including sense on a first channel using at least a first electrode at a first location and sense on a second channel using at least a second electrode at a second location, the controller is configured to sample the sensed evoked potentials on each of the first and second channels detect at least one feature on each of the channels, and automatically determine that the at least one feature on the first channel corresponds to the at least one feature on the second channel with an expected propagation delay from the first location to the second location.

3. The system (100, 200) of any one of claims 1-2, wherein the controller (311) is configured to automatically set an initial sampling window for the electrical therapy.

4. The system (100, 200) of any one of claims 1-3, wherein the controller (311) is configured to automatically adjust the sampling window during the course of the electrical therapy.

5. The system (100, 200) of claim 4, wherein the controller (311) is configured to automatically adjust the sampling window to avoid a stimulation artifact (834, 934) from interfering with detecting the at least one feature.

6. The system (100, 200) of claim 4, wherein the controller is configured to automatically detect at least one of a minimum or a maximum in the sensed evoked potentials.

7. The system (100, 200) of claim 6, wherein the controller is configured to determine whether the at least one feature occurs before an expected period of time or a number of samples after the stimulation pulse and whether another feature occurs during the expected period of time or the number of samples after the stimulation pulse, and wherein the controller is configured to delay the beginning of the sampling window after the stimulation pulse.

8. The system (100, 200) of claim 6, wherein the controller (311) is configured to determine that the at least one feature occurs before and does not occur during the expected period of time or sample range after the stimulation pulse, and before automatically adjusting the sampling window reassess whether the at least one feature occurs before and does not occur during the expected period of time or sample range after the stimulation pulse.

9. The system (100, 200) of any one of claims 1-8, wherein the controller (311) is configured to find an expected peak and an expected trough in the sensed evoked potentials, and use the expected peak and the expected trough as a reference to move the sampling window to avoid the stimulation artifact (834, 934).

10. The system (100, 200) of any one of claims 1-9, wherein the at least one feature includes at least one of a curve length or an area under a curve between two points in sensed evoked potentials.

11. The system (100, 200) of any one of claims 1-10, wherein the sensed evoked potentials include at least one feature-time association, and the controller (311) is configured to automatically define the sampling window based on the at least one feature-time association, wherein the feature-time association for a time window includes at least one of:
an area under the curve for the time window,
a curve length for the time window,
a range for the time window,
an oscillation frequency within the time window,
a rate of decay in amplitude of the peaks in the evoked response within the time window,
a difference of any two positive and negative peak magnitudes within the time window, or
a change in at least one feature value within the time window with respect to a baseline feature value.

12. The system (100, 200) of any one of claims 1-11, wherein the controller (311) is configured to preset a sampling window based on an estimated conduction velocity and a distance between the stimulation electrode and sensing electrode.

13. The system (100, 200) of any one of claims 1-12, wherein the sensing circuitry (310) is configured to sense on at least two channels, including sense on a first channel using at least a first electrode at a first location and sense on a second channel using at least a second electrode at a second location, wherein the controller is configured to sample the sensed evoked potentials on each of the first and second channels, detect at least one feature on each of the channels, and determine that the at least one feature on the first channel corresponds to the at least one feature on the second channel with an expected propagation delay from the first location to the second location, and set the sampling window for the first channel based on the detected at least one feature in the first channel and set the sampling window for the second channel based on the detected at least one feature in the second channel.

## Patentansprüche

1. System (100, 200), aufweisend:
einen Stimulator (102, 302), der mit zumindest einer Stimulationselektrode (101, 301) wirkverbunden ist und konfiguriert ist, eine elektrische Wellenform für eine elektrische Therapie unter Verwendung der zumindest einen Stimulationselektrode abzugeben;
eine Erfassungsschaltung (310), die mit zumindest einer Erfassungselektrode wirkverbunden ist und konfiguriert ist, elektrische Potentiale zu erfassen, die durch die elektrische Wellenform bewirkt werden, um erfasste bewirkte Signale bereitzustellen;
eine Steuerung (311), die mit dem Stimulator und der Erfassungsschaltung (310) wirkverbunden ist, wobei die Steuerung konfiguriert ist zum
automatischen Definieren eines Abtastfensters;
Abtasten der erfassten bewirkten Potentiale während des Abtastfensters, um Abtastwerte bereitzustellen;
Detektieren zumindest eines Merkmals aus den Abtastwerten; und
automatischen Bereitstellen einer Rückkopplung für eine Regelung der elektrischen Therapie im geschlossenen Regelkreis auf Grundlage des zumindest einen Merkmals, wobei die Steuerung (311) konfiguriert ist, das Abtastfenster automatisch zu definieren durch:
Verwenden einer Nachschlagetabelle, um das Abtastfenster automatisch auf Grundlage einer geschätzten Leitungsgeschwindigkeit für die bewirkten Potentiale und eines Abstands zwischen der Stimulationselektrode und der Erfassungselektrode zu definieren, wobei die Nachschlagetabelle einen Beginn und ein Ende des Abtastfensters für eine ausgewählte Stimulationselektrode und eine ausgewählte Erfassungselektrode aus einer Mehrzahl von Elektroden identifiziert.

2. System (100, 200) nach Anspruch 1, wobei die Erfassungsschaltung (310) konfiguriert ist, auf zumindest zwei Kanälen zu erfassen, einschließlich des Erfassens auf einem ersten Kanal unter Verwendung zumindest einer ersten Elektrode an einem ersten Ort und des Erfassens auf einem zweiten Kanal unter Verwendung zumindest einer zweiten Elektrode an einem zweiten Ort, wobei die Steuerung konfiguriert ist, die erfassten bewirkten Potentiale auf jedem des ersten und zweiten Kanals abzutasten, zumindest ein Merkmal auf jedem der Kanäle zu detektieren und automatisch zu bestimmen, dass das zumindest eine Merkmal auf dem ersten Kanal dem zumindest einen Merkmal auf dem zweiten Kanal mit einer erwarteten Ausbreitungsverzögerung von dem ersten Ort zu dem zweiten Ort entspricht.

3. System (100, 200) nach einem der Ansprüche 1 bis 2, wobei die Steuerung (311) konfiguriert ist, ein anfängliches Abtastfenster für die elektrische Therapie automatisch festzulegen.

4. System (100, 200) nach einem der Ansprüche 1 bis 3, wobei die Steuerung (311) konfiguriert ist, das Abtastfenster im Verlauf der elektrischen Therapie automatisch anzupassen.

5. System (100, 200) nach Anspruch 4, wobei die Steuerung (311) konfiguriert ist, das Abtastfenster automatisch anzupassen, um zu vermeiden, dass ein Stimulationsartefakt (834, 934) das Detektieren des zumindest einen Merkmals stört.

6. System (100, 200) nach Anspruch 4, wobei die Steuerung konfiguriert ist, ein Minimum und/oder ein Maximum in den erfassten bewirkten Potentialen automatisch zu detektieren.

7. System (100, 200) nach Anspruch 6, wobei die Steuerung konfiguriert ist zu bestimmen, ob das zumindest eine Merkmal vor einem erwarteten Zeitraum oder einer Anzahl von Abtastwerten nach dem Stimulationsimpuls auftritt und ob ein anderes Merkmal während des erwarteten Zeitraums oder der Anzahl von Abtastwerten nach dem Stimulationsimpuls auftritt, und wobei die Steuerung konfiguriert ist, den Beginn des Abtastfensters nach dem Stimulationsimpuls zu verzögern.

8. System (100, 200) nach Anspruch 6, wobei die Steuerung (311) konfiguriert ist zu bestimmen, dass das zumindest eine Merkmal vor und nicht während des erwarteten Zeitraums oder Abtastbereichs nach dem Stimulationsimpuls auftritt , und vor dem automatischen Anpassen des Abtastfensters erneut zu beurteilen, ob das zumindest eine Merkmal vor und nicht während des erwarteten Zeitraums oder Abtastbereichs nach dem Stimulationsimpuls auftritt.

9. System (100, 200) nach einem der Ansprüche 1 bis 8, wobei die Steuerung (311) konfiguriert ist, einen erwarteten Spitzenwert und ein erwartetes Wellental in den erfassten bewirkten Potentialen zu finden und den erwarteten Spitzenwert und das erwartete Wellental als Referenz zu verwenden, um das Abtastfenster zu verschieben, um das Stimulationsartefakt (834, 934) zu vermeiden.

10. System (100, 200) nach einem der Ansprüche 1 bis 9, wobei das zumindest eine Merkmal zumindest eines von einer Kurvenlänge oder einer Fläche unter einer Kurve zwischen zwei Punkten in erfassten bewirkten Potentialen aufweist.

11. System (100, 200) nach einem der Ansprüche 1 bis 10, wobei die erfassten bewirkten Potentiale zumindest eine Merkmal-Zeit-Zuordnung aufweisen und die Steuerung (311) konfiguriert ist, das Abtastfenster auf Grundlage der zumindest einen Merkmal-Zeit-Zuordnung automatisch zu definieren, wobei die Merkmal-Zeit-Zuordnung für ein Zeitfenster aufweist:
eine Fläche unter der Kurve für das Zeitfenster, und/oder
eine Kurvenlänge für das Zeitfenster, und/oder
einen Bereich für das Zeitfenster, und/oder
eine Oszillationsfrequenz innerhalb des Zeitfensters, und/oder
eine Abklingrate in der Amplitude der Spitzenwerte in der bewirkten Antwort innerhalb des Zeitfensters, und/oder
eine Differenz beliebiger zwei positiver und negativer Spitzenwertgrößen innerhalb des Zeitfensters, und/oder
eine Änderung zumindest eines Merkmalswerts innerhalb des Zeitfensters in Bezug auf einen Basislinien-Merkmalswert.

12. System (100, 200) nach einem der Ansprüche 1 bis 11, wobei die Steuerung (311) konfiguriert ist, ein Abtastfenster auf Grundlage einer geschätzten Leitungsgeschwindigkeit und eines Abstands zwischen der Stimulationselektrode und der Erfassungselektrode voreinzustellen.

13. System (100, 200) nach einem der Ansprüche 1 bis 12, wobei die Erfassungsschaltung (310) konfiguriert ist, auf zumindest zwei Kanälen zu erfassen, einschließlich des Erfassens auf einem ersten Kanal unter Verwendung zumindest einer ersten Elektrode an einem ersten Ort und des Erfassens auf einem zweiten Kanal unter Verwendung zumindest einer zweiten Elektrode an einem zweiten Ort, wobei die Steuerung konfiguriert ist, die erfassten bewirkten Potentiale auf jedem des ersten und zweiten Kanals abzutasten, zumindest ein Merkmal auf jedem der Kanäle zu detektieren und zu bestimmen, dass das zumindest eine Merkmal auf dem ersten Kanal dem zumindest einen Merkmal auf dem zweiten Kanal mit einer erwarteten Ausbreitungsverzögerung von dem ersten Ort zu dem zweiten Ort entspricht, und das Abtastfenster für den ersten Kanal auf Grundlage des detektierten zumindest einen Merkmals in dem ersten Kanal festzulegen und das Abtastfenster für den zweiten Kanal auf Grundlage des detektierten zumindest einen Merkmals in dem zweiten Kanal festzulegen.

## Revendications

1. Système (100, 200), comprenant :
un stimulateur (102, 302) connecté fonctionnellement à au moins une électrode de stimulation (101, 301), et configuré pour délivrer une forme d'onde électrique pour une thérapie électrique à l'aide de l'au moins une électrode de stimulation ;
un ensemble de circuits de détection (310) connecté fonctionnellement à au moins une électrode de détection, et configuré pour détecter des potentiels électriques évoqués par la forme d'onde électrique afin de fournir des signaux évoqués détectés ;
un contrôleur (311) connecté fonctionnellement au stimulateur et à l'ensemble de circuits de détection (310), dans lequel le contrôleur est configuré pour
définir automatiquement une fenêtre d'échantillonnage ;
échantillonner les potentiels évoqués détectés pendant la fenêtre d'échantillonnage afin de fournir des valeurs échantillonnées ;
détecter au moins une caractéristique à partir des valeurs échantillonnées ; et
fournir automatiquement une rétroaction pour une commande en boucle fermée de la thérapie électrique sur la base de l'au moins une caractéristique,
dans lequel le contrôleur (311) est configuré pour définir automatiquement la fenêtre d'échantillonnage en :
utilisant une table de correspondance pour définir automatiquement la fenêtre d'échantillonnage sur la base d'une vitesse de conduction estimée pour les potentiels évoqués et d'une distance entre l'électrode de stimulation et l'électrode de détection, la table de correspondance identifiant un début et une fin de la fenêtre d'échantillonnage pour une électrode de stimulation sélectionnée et une électrode de détection sélectionnée parmi une pluralité d'électrodes.

2. Système (100, 200) selon la revendication 1, dans lequel l'ensemble de circuits de détection (310) est configuré pour effectuer une détection sur au moins deux canaux, comprenant une détection sur un premier canal à l'aide d'au moins une première électrode à un premier emplacement et une détection sur un deuxième canal à l'aide d'au moins une deuxième électrode à un deuxième emplacement, le contrôleur étant configuré pour échantillonner les potentiels évoqués détectés sur chacun des premier et deuxième canaux, détecter au moins une caractéristique sur chacun des canaux, et déterminer automatiquement que l'au moins une caractéristique sur le premier canal correspond à l'au moins une caractéristique sur le deuxième canal avec un retard de propagation attendu du premier emplacement au deuxième emplacement.

3. Système (100, 200) selon l'une quelconque des revendications 1 à 2, dans lequel le contrôleur (311) est configuré pour définir automatiquement une fenêtre d'échantillonnage initiale pour la thérapie électrique.

4. Système (100, 200) selon l'une quelconque des revendications 1 à 3, dans lequel le contrôleur (311) est configuré pour ajuster automatiquement la fenêtre d'échantillonnage au cours de la thérapie électrique.

5. Système (100, 200) selon la revendication 4, dans lequel le contrôleur (311) est configuré pour ajuster automatiquement la fenêtre d'échantillonnage afin d'éviter qu'un artefact de stimulation (834, 934) n'interfère avec la détection de l'au moins une caractéristique.

6. Système (100, 200) selon la revendication 4, dans lequel le contrôleur est configuré pour détecter automatiquement au moins l'un d'un minimum ou d'un maximum dans les potentiels évoqués détectés.

7. Système (100, 200) selon la revendication 6, dans lequel le contrôleur est configuré pour déterminer si l'au moins une caractéristique survient avant une période de temps attendue ou un nombre d'échantillons après l'impulsion de stimulation et si une autre caractéristique survient pendant la période de temps attendue ou le nombre d'échantillons après l'impulsion de stimulation, et dans lequel le contrôleur est configuré pour retarder le début de la fenêtre d'échantillonnage après l'impulsion de stimulation.

8. Système (100, 200) selon la revendication 6, dans lequel le contrôleur (311) est configuré pour déterminer que l'au moins une caractéristique survient avant et ne survient pas pendant la période de temps attendue ou la plage d'échantillons après l'impulsion de stimulation, et, avant l'ajustement automatique de la fenêtre d'échantillonnage, réévaluer si l'au moins une caractéristique survient avant et ne survient pas pendant la période de temps attendue ou la plage d'échantillons après l'impulsion de stimulation.

9. Système (100, 200) selon l'une quelconque des revendications 1 à 8, dans lequel le contrôleur (311) est configuré pour trouver un pic attendu et un creux attendu dans les potentiels évoqués détectés, et utiliser le pic attendu et le creux attendu comme référence pour déplacer la fenêtre d'échantillonnage afin d'éviter l'artefact de stimulation (834, 934).

10. Système (100, 200) selon l'une quelconque des revendications 1 à 9, dans lequel l'au moins une caractéristique comprend au moins l'une d'une longueur de courbe ou d'une aire sous une courbe entre deux points dans les potentiels évoqués détectés.

11. Système (100, 200) selon l'une quelconque des revendications 1 à 10, dans lequel les potentiels évoqués détectés comprennent au moins une association caractéristique-temps, et le contrôleur (311) est configuré pour définir automatiquement la fenêtre d'échantillonnage sur la base de l'au moins une association caractéristique-temps, dans lequel l'association caractéristique-temps pour une fenêtre temporelle comprend au moins l'un des éléments suivants :
une aire sous la courbe pour la fenêtre temporelle,
une longueur de courbe pour la fenêtre temporelle,
une plage pour la fenêtre temporelle,
une fréquence d'oscillation dans la fenêtre temporelle,
un taux de décroissance de l'amplitude des pics dans la réponse évoquée dans la fenêtre temporelle,
une différence entre deux amplitudes de pics positifs et négatifs quelconques dans la fenêtre temporelle, ou
une variation d'au moins une valeur de caractéristique dans la fenêtre temporelle par rapport à une valeur de caractéristique de référence.

12. Système (100, 200) selon l'une quelconque des revendications 1 à 11, dans lequel le contrôleur (311) est configuré pour prédéfinir une fenêtre d'échantillonnage sur la base d'une vitesse de conduction estimée et d'une distance entre l'électrode de stimulation et l'électrode de détection.

13. Système (100, 200) selon l'une quelconque des revendications 1 à 12, dans lequel l'ensemble de circuits de détection (310) est configuré pour effectuer une détection sur au moins deux canaux, comprenant une détection sur un premier canal à l'aide d'au moins une première électrode à un premier emplacement, et une détection sur un deuxième canal à l'aide d'au moins une deuxième électrode à un deuxième emplacement, dans lequel le contrôleur est configuré pour échantillonner les potentiels évoqués détectés sur chacun des premier et deuxième canaux, détecter au moins une caractéristique sur chacun des canaux, et déterminer que l'au moins une caractéristique sur le premier canal correspondent à l'au moins une caractéristique sur le deuxième canal avec un retard de propagation attendu du premier emplacement au deuxième emplacement, et définir la fenêtre d'échantillonnage pour le premier canal sur la base de l'au moins une caractéristique détectée dans le premier canal, et définir la fenêtre d'échantillonnage pour le deuxième canal sur la base de l'au moins une caractéristique détectée dans le deuxième canal.
